**Europäisches Patentamt**

**European Patent Office**

**Office européen des brevets**

(19)

(11) Publication number: **0 182 295**
**A2**

(12) # EUROPEAN PATENT APPLICATION

(21) Application number: **85114490.7**

(22) Date of filing: **14.11.85**

(51) Int. Cl.⁴: **A 61 K 7/035**, C 08 B 30/12

(30) Priority: **15.11.84 US 671712**

(43) Date of publication of application: **28.05.86 Bulletin 86/22**

(84) Designated Contracting States: **BE CH DE FR GB IT LI NL**

(71) Applicant: **CPC INTERNATIONAL INC., International Plaza P.O. Box 8000, Englewood Cliffs New Jersey 07632 (US)**

(72) Inventor: **Adams, Heidi, 2114 Wesley, Berwyn Illinois 60402 (US)**
Inventor: **Kochan, David A., 1534-77th Street, Darien Illinois 60559 (US)**
Inventor: **Zobel, Henry F., 1105 Belair Drive, Darien Illinois 60559 (US)**

(74) Representative: **Lederer, Franz, Dr. et al, Patentanwälte Dr. Franz Lederer Dipl.-Ing. Reiner F. Meyer-Roxlau Lucile-Grahn-Strasse 22, D-8000 München 80 (DE)**

(54) Dusting powder compositions containing modified starch.

(57) A dusting powder composition with improved moisture sorbing capacity which contains a modified starch. A method for preparation of the powder whereby starch, water and surfactant are heated in a fluidizing apparatus.

3334

# DUSTING POWDER COMPOSITIONS CONTAINING MODIFIED STARCH

This invention relates to dusting powder compositions and a process for their manufacture. The compositions contain modified starch with better water sorbing capacity than native starch.

There is today a need for a material which can serve as a base for body dusting powders. Generally, a widely-used constituent of such formulations has been talc. However, talc has come under increasing medical scrutiny as possibly containing a carcinogenic substance, asbestos. Pure mineral talc is a group of hydrous magnesium silicates. Unlike pure mineral talc, commercial talc has varying compositions depending on the source and method of production. It has been postulated that some of the commercial talc may be contaminated with asbestos.

Commercial native corn starch has been used to some extent as a replacement for talc in body dusting powders, particularly, in baby powder. However, unmodified corn starch is not completely satisfactory for these purposes. When it absorbs moisture during use, the granules become tacky and adhere to each other. This results in a gummy, pasty feel on the skin and the moistened starch can form rolls or beads of starch rather than spreading evenly.

Numerous attempts have been made to prepare modified starches that would have good tactile properties even in the presence of moisture. One such product was disclosed by Krisan in U.S. Patent 2,614,945. In this disclosure, starch granules were coated with a thin layer of an insoluble inorganic salt. In U.S. Patent 2,852,404, Satterthwaite disclosed coating starch granules with an aluminum soap. Other workers have proposed making inhibited starches, such as cross-linked starches or cross-linked esters for use as dusting powders. While such procedures do give compositions which are more hydrophobic than native starch, the products generally have poor tactile properties or do not sorb sufficient moisture to make them suitable for many dusting starch uses. In particular, they are not suitable as body dusting starches.

We have now discovered a specially modified starch product which has good tactile properties when used as a body dusting starch and which sorbs more water than either talc or native starch.

In accordance with this invention, there is provided a dusting powder composition comprising an intimate mixture of a modified starch, having a moisture sorbing capacity of from about 1.0 gram to about 1.6 grams of water per gram of starch, and from about 0.25% to about 2% by weight of the starch of a surfactant.

Also provided, in accordance with this invention, is a process for preparing a dusting powder, which comprises forming an intimate mixture comprising from about 60% to about 85% by weight of starch on a dry solids basis, from about 15% to about 40% by weight of water and from about 0.25% to about 2.0% by weight of the starch of a surfactant, fluidizing said mixture with a fluidizing gas in a fluidizing apparatus, heating said mixture in the fluidizing apparatus at a temperature of from about $100^{\circ}C$ to about $155^{\circ}C$ for a sufficient time to give a dusting powder having a moisture sorption capacity of from about 1.0 gram to about 1.6 grams of water per gram of powder, and recovering the dusting powder from the fluidizing apparatus.

-3-

0182295

The improved dusting powder compositions of this invention comprises a mixture of a modified starch with a surfactant. The modified starch has greater ability to sorb water than does its unmodified parent. In general, the dusting powder compositions of this invention sorb up to three times as much water as the same weight of talc, and up to two times as much water as the same weight of unmodified starch under the same conditions of water sorption.

The dusting powder compositions of this invention can be prepared by forming an intimate mixture of a modified starch with a surfactant. Alternatively, the dusting powder compositions of this invention can be prepared by mixing an unmodified starch with a surfactant and then treating the mixture under conditions whereby the starch is modified.

In accordance with one embodiment of this invention, an unmodified starch is mixed with a limited amount of water and the mixture is heated in a fluidizing apparatus. Heating is conducted under conditions which modify the starch until it will sorb from about 1.0 gram to about 1.6 grams of water per gram of the modified starch.

-4-

The fluidizing apparatus used for this process should be one that gives good fluidization of the starch. A suitable fluidizing apparatus for this purpose is described in U.S. Patent 3,967,975, and its modification is described in U.S. Patent 4,237,619.

Various native starches can be used in the practice of this invention. These include corn, tapioca, wheat, potato, rice, sago, grain sorghum and waxy maize starches.

The amount of water mixed with the starch before it is heated in the fluidizing apparatus can vary from about 15% to about 40% by weight of the starch-water mixture. The preferred amount of water is from about 25% to about 35% by weight of the mixture. The temperature of the mixture in the fluidizing apparatus is maintained between about 100°C and about 155°C. Preferably, the temperature of the mixture is maintained between about 105°C and 135°C.

According to another embodiment of this invention, the starch is first blended with a mixture of water and a surfactant before it is heated in the fluidizing apparatus. Various common surfactants can be used for this purpose. When the dusting powder is to be used in food applications, it is necessary to use a surfactant that is acceptable for food use. Such surfactants are also preferred when the powder is used as a

-5-

0182295

body dusting powder. Suitable surfactants are glycerides, such as a mono or diglyceride of a fatty acid. Examples of most preferred surfactants for these purposes are glycerol monostearate and sodium stearoyl lactylate. Other suitable surfactants containing a fatty acid moiety are acceptable in the process of this invention. These include palmitic or stearic fatty acids, D-glucose 3-stearate, methyl alpha-D-glucoside 6-stearate, sucrose monostearate, sorbitan tetrastearate, stearoyl-2-lactylate and alkali metal salts thereof.

The amount of surfactant employed is from about 0.25% to about 2.0% by weight of the starch. Preferably, from about 0.5% to about 1.0% by weight of the starch is used.

Another modified starch which can sorb more moisture than native starch is high amylose starch. Native cereal starches, such as corn starch, contain about 27% amylose. Native strains of corn have been hybridized to give new strains which produce modified starches with much higher percentages of amylose. We have discovered that if such modified starches contain at least about 50% amylose, they will show improved moisture sorption capabilities. Modified starches containing about 70% amylose have even higher moisture sorbing capabilites. When such starches are used to prepare the dusting powder compositions of this invention, they are blended with from about 0.25% to about 2%, preferably, from about 0.5% to about 1% by weight of the starch of a surfactant.

-6-

0182295

The starch products of this invention can be used as replacement for talc in body dusting powders and in other applications where dusting powders are used. It is often the practice in the formulation of such powders to include some additives to make the powder more acceptable for a particular use. These additives include such materials as fragrances and flow promoters. Such additives may be used with the starches of the present invention and do not interfere with the improved water sorption properties of the starches when used in relatively small concentrations.

The following examples illustrate the invention. It is to be understood that the examples are illustrative only and do not intend to limit the invention in any way.

### EXAMPLE 1

A mixture of 66% by weight of starch on a dry solids basis, 34% water and 0.33% glycerol monostearate was mixed thoroughly in a blender. The glycerol monostearate was added to the starch as a dispersion in hot water. The mixture was then heated in a fluidizing apparatus containing a lower-agitated section, an intermediate section containing 7 tubes and an upper-agitated section. This apparatus is described in U.S. Patent 3,967,975. The mixture was added to the apparatus at a rate of 2.27 kg/min. The jacket surrounding the tubes was heated with steam to raise the internal

-7-

temperature to 107°C and the mixture was fluidized with air passing through the apparatus at a rate of about 14 liters per second. The product was removed from the apparatus after a mean residence time of about 15 minutes and milled to pass through a No. 200 U.S. Standard sieve. The product showed a water sorption of 1.3 grams per gram of starch.

Water sorption is determined by weighing accurately about 2 grams of solid in a 40-cc Gooch crucible (Coors) having a perforated bottom covered with a moist Whatman glass microfiber filter. The crucible is covered with a watch glass and set in a petri dish containing a 1% NaCl solution. The depth of the solution in the dish is maintained at about 8 mm. After 24 hours, the crucible is removed, wiped dry and weighed to determine the amount of water sorbed by the solid. For comparison purposes, it was determined that native corn starch sorbs about 0.7 gram of water, and talc sorbs about 0.4 gram of water in this test.

When the procedure of Example 1 was followed using larger and smaller amounts of glycerol monostearate, products with comparable water sorption were obtained. The products with lower amounts of glycerol monostearate showed less smoothness and lubricity when used as a body dusting powder. Whereas, the products with larger amounts of glycerol monostearate showed even more smoothness and lubricity than the product of Example 1.

## EXAMPLE 2

The general process of Example 1 was repeated without the addition of the surfactant to determine the conditions under which starches with good moisture sorption were produced. The various conditions under which these runs were made and the moisture sorption of the products are given in Table I. They show that starch containing various amounts of moisture can be treated in the fludizing apparatus to give products with improved moisture sorption over that of the native starch.

### TABLE I

| | Reaction Conditions | | | Product |
| Run No. | % $H_2O$ | Feed Rate (kg/min) | Temp ($^{o}C$) | Water Sorption (g $H_2O$/g Starch) |
|---|---|---|---|---|
| 1 | 30.3 | 2.27 | 115-118 | 1.0 |
| 2 | 32.4 | 1.59 | 135 | 1.4 |
| 3 | 35 | 2.27 | 102 | 1.5 |

Various batches of high amylose corn starch were tested for water sorption by the test used in Example 1.  The portion of the starch which would pass through a No. 200 U.S. Standard sieve was employed.  The results given in Table II show that these modified starches give better water sorption than does the starch from native corn.  When these starches are mixed with from 0.25 to 2.0% of the surfactant, they exhibit tactile properties making them suitable as body dusting starches.

TABLE II

| Batch No. | % Amylose in Starch | Water Sorption (g $H_2O$/g Starch) |
|---|---|---|
| 1 | 70 | 1.26 |
| 2 | 70 | 1.43 |
| 3 | 70 | 1.15 |
| 4 | 57 | 1.13 |

Thus, it is apparent that there has been provided, in accordance with the invention, dusting powder starches and a process for their preparation that fully satisfy the objects, aims and advantages set forth above.  While the invention has been described in conjunction with specific embodiments thereof, it is evident that many alternatives, modifications, and variations will be apparent to those skilled in the art in light of the foregoing description.  Accordingly, it is intended to include all such alternatives, modifications, and variations as set forth within the spirit and scope of the appended claims.

Claims

1. A dusting powder composition comprising an intimate mixture of a modified starch and from about 0.25% to about 2% by weight of the starch of a surfactant, characterized in that the modified starch obtained by heating a starch-water mixture containing from about 15% to about 40% by weight of water, in a fluidizing apparatus maintained between about 100°C and about 155°C, for a sufficient time to give a modified starch having a moisture capacity of from about 1.0 gram to about 1.6 grams of water per gram of starch.

2. A dusting powder composition comprising an intimate mixture of a starch and from about 0.25% to about 2% by weight of the starch of a surfactant characterized in that the modified starch is a high amylose corn starch comprising at least about 50% by weight of amylose.

3. The composition of claim 2 characterized in that the high amylose corn starch comprises at least about 70% by weight of amylose.

4. A process for preparing a dusting powder characterized by the steps of forming an intimate mixture comprising from about 60% to about 85% by weight of starch from

-12-

about 15% to about 40% by weight of water and from about 0.25% to about 2.0% by weight of the starch of a surfactant, fluidizing said mixture with a fluidizing gas in a fluidizing apparatus, heating said mixture in the fluidizing apparatus at a temperature of from about 100°C to about 155°C for a sufficient time to give a dusting powder having a moisture sorption capacity of from about 1.0 gram to about 1.6 grams of water per gram of powder, and recovering the dusting powder from the fluidizing apparatus.

5.   The process of claim 4 characterized in that the starch is corn starch.

6.   The process of claim 5 characterized in that the amount of water in the mixture is from about 25% to about 35% of the weight of the mixture.

7.   The process of claim 6 characterized in that the temperature of the fluidizing apparatus is between about 105°C and 135°C.

8.   The process of claim 7 characterized in that the amount of surfactant is between 0.5% and 1% by weight of the starch.

-13-

9. The process of claim 4 characterized in that nominal residence time of the starch in the fluidizing apparatus is about 15 minutes.

10. The process of claim 4 characterized in that the surfactant is glycerol monostearate.